# EUROPEAN PATENT SPECIFICATION

(11) **EP 4 228 724 B1**
(45) Date of publication and mention of the grant of the patent: **01.05.2024**
(21) Application number: 21787482.5
(22) Date of filing: 15.10.2021
(51) Int. Cl.: A61M 11/00, A61M 15/00, A61M 15/02, B05B 12/08

(54) **METHOD FOR DETECTING THE PRESENCE OF LIQUID IN A VIBRATING MEMBRANE NEBULIZER**
VERFAHREN ZUR DETEKTION DER ANWESENHEIT VON FLÜSSIGKEIT IN EINEM VERNEBLER MIT VIBRIERENDER MEMBRAN
PROCÉDÉ DE DÉTECTION DE LA PRÉSENCE DE LIQUIDE DANS UN NÉBULISEUR À MEMBRANE VIBRANTE

(30) Priority: 16.10.2020 EP 20202253
(43) Date of publication of application: 23.08.2023
(73) Proprietor: Vectura Delivery Devices Limited, Chippenham, Wiltshire SN14 6FH (GB)
(72) Inventor: HUBER, Martin, Cambridge Cambridgeshire CB4 0GZ (GB); FREY, Manual, Cambridge Cambridgeshire CB4 0GZ (GB); LACHER, Maximilian, Cambridge Cambridgeshire CB4 0GZ (GB); SCHWENDNER, Sebastian, Cambridge Cambridgeshire CB4 0GZ (GB); KOLB, Tobias, Cambridge Cambridgeshire CB4 0GZ (GB); HOFFMANN, Tobias, Cambridge Cambridgeshire CB4 0GZ (GB); WEISER, Yannic, Cambridge Cambridgeshire CB4 0GZ (GB); TRINKHAUS, Jonas, Cambridge Cambridgeshire CB4 0GZ (GB)
(74) Representative: Clarke, Christopher John
(86) International application number: PCT/EP2021/078627
(87) International publication number: WO 2022/079249

(56) References cited:
- EP-A1- 2 724 741
- WO-A1-2015/091356
- WO-A1-2017/199215
- WO-A1-2020/120665
- US-A- 5 906 202
- US-A1- 2006 102 178
- US-A1- 2009 240 192
- US-A1- 2020 276 398

## Description

### Technical Field of the Invention

The present invention relates to a vibrating membrane nebulizer, and in particular to a method for detecting the presence of liquid in contact with the membrane.

### Background to the Invention

Aerosols for medical inhalation therapy generally comprise an active ingredient dissolved or suspended in an aerosolisable liquid (often water). A homogeneous distribution of aerosol droplets with a droplet size of around 5 µm is required in order to reach deep into the lungs.

Vibrating membrane nebulizers are one type of device for producing such aerosols. These devices comprise a vibrator, such as piezoelectric element, which is excited at ultrasonic frequencies in order to induce vibration of a membrane (sometimes called a mesh or aperture plate). The membrane has a large number of holes which typically have a diameter of 1 µm to 10 µm. A reservoir supplies the liquid drug formulation to the membrane. Vibration of the membrane leads to the formation and emission of aerosol droplets of the liquid through the holes.

Continuing to operate a vibrating membrane nebulizer after the liquid reservoir has been emptied can cause the membrane to crack or break. Therefore, it is important to be able to reliably detect the presence of liquid in the reservoir / or in contact with the membrane. When the nebulizer detects that the liquid has been used up, it can turn off the vibrator automatically and / or indicate the end of the therapy session to the patient.

One approach is simply to measure the presence or amount of liquid in the reservoir. For example, US2006/0255174 discloses a nebulizer in which the amount of liquid in the reservoir is sensed by a piezoelectric sensor, an optical sensor, a conductivity sensor or a strain gauge. However, this requires contact between the sensor and the liquid, which can present problems.

An alternative approach exploits the fact that the vibration characteristics of the membrane (e.g. resonant frequency, power consumption etc.) typically are quite different when the membrane is in contact with liquid compared to when it is dry. For example, US2006/0102172 discloses a nebulizer which determines whether liquid is present or not by comparing the detected value of an electrical parameter (such as the current to the piezoelectric element) with a stored value. WO2015/091356 discloses an aerosol delivery device with a membrane, a vibrator and a fluid reservoir. The device operates the vibrator at a plurality of different vibration frequencies, and a sensor measures an electrical parameter of the vibrator at each frequency. The device detects the presence of fluid in contact with the membrane and/or in the fluid reservoir on the basis of the dependence of the values of the electrical parameter on the frequency. However, variations between different membranes and / or changes in the membrane over its lifetime can result in these methods failing to detect that the membrane is dry. If the nebulizer does not correctly determine whether liquid is present in the reservoir, it could continue to vibrate the membrane after the liquid has been used up, or it could turn the vibrator off while liquid still remains.

Thus there remains a need for improved methods for reliably detecting whether the liquid is present in the reservoir and / or in contact with the membrane.

### Brief Description of the Invention

The present inventors have identified an improved method for determining when the liquid to be aerosolized has been used up. In particular, they recognized that light scattering can be used to reliably detect the absence of liquid in contact with the membrane, by detecting the absence of aerosol within the nebulizer. Accordingly, in a first aspect, the present invention provides an inhalation device comprising:
- a channel having an air inlet opening and an aerosol outlet opening,
- an aerosol generator comprising a vibrator and a membrane,
- a reservoir for liquid to be aerosolized which is fluidically connected to the membrane,
- an optical sensor for detecting the presence of aerosol within the channel,
- a controller which is configured (i) to provide a driver signal to operate the vibrator so that the membrane vibrates and generates an aerosol in the channel; (ii) to receive an output signal from the sensor; (iii) to determine whether aerosol is present in the channel on the basis of the output signal; and (iv) to stop operating the vibrator if it determines that no aerosol is present.

The aerosol generator may comprise a support member on which the vibrator and / or the membrane are mounted. The vibrator may be an annular piezoelectric element. The support member may be a transducer in the form of a hollow tubular portion having a flange at or close to a first end onto which the piezoelectric element is attached, and a second end into or onto which the membrane is mounted. Alternatively, the support member may comprise an essentially planar annulus or disk; in particular the membrane and / or the piezoelectric element may be mounted on opposite sides of the support member.

The optical sensor may operate in the infrared region of the electromagnetic spectrum. The optical sensor may comprise an emitter and a detector that are located on opposite sides of the channel so that the detector detects light from the emitter that is transmitted through the aerosol across the channel.

The controller is configured (i) to modulate the amplitude of the driver signal at a frequency of from 1 to 100 Hz, (ii) to demodulate the output signal at the same frequency; and (iii) to determine whether aerosol is present in the channel on the basis of the demodulated output signal. The modulation frequency may be from 2 to 70 Hz or 3 to 55 Hz, or 5 to 40 Hz, for example about 10, 20 or 30Hz. The controller may be configured to modulate the amplitude of the vibration of the membrane with a sinusoidal, saw-tooth or square wave.

The controller may be configured (i) to periodically perform a scan to determine a resonant frequency of the aerosol generator and / or the membrane, during which the membrane generates aerosol at a reduced rate; (ii) to demodulate the output signal at a scanning frequency, such as 2Hz, that corresponds to the period between scans, such as 0.5s; and (iii) to determine whether aerosol is present in the channel on the basis of the demodulated output signal.

The controller may be configured to determine the phase difference between the driver signal and the output signal, and thereby to determine the velocity of the aerosol as it passes the optical sensor.

The inhalation device may further comprise a variable flow restrictor which restricts the flow rate of the air and aerosol to a maximum flow rate of about 20 Litres /minute or 18 L/min, such as about 15 L/min. The inhalation device may further comprise a pressure sensor for measuring pressure in the channel, and a signalling device capable of emitting light of varying intensity, and the controller may be configured (i) to receive a signal representing the measured pressure and (ii) to cause the signalling device to emit light of lower intensity the further the measured pressure deviates from a target pressure.

The channel may have an internal volume between the membrane and the optical sensor of less than 5 cm³ for example from 0.5 to 3 cm³ or from 1 to 2 cm³, such as about 1.5 cm³. The channel may be, be part of or comprise, a component that is removable from the rest of the device.

In a second aspect, the present invention provides a method of operating an inhalation device according to the first aspect of the invention, the method comprising:
a) providing a driver signal to operate the vibrator so that the membrane vibrates and generates an aerosol in the channel;
b) receiving an output signal from the optical sensor;
c) determining whether aerosol is present in the channel on the basis of the output signal; and
d) ceasing to operate the vibrator if it is determined in step c) that no aerosol is present.

The method further comprises: in step a), modulating the amplitude of the driver signal at a frequency of from 1 to 100 Hz; in step b), demodulating the output signal at the same frequency; and in step c), determining whether aerosol is present in the channel on the basis of the demodulated output signal. The modulation frequency may be from 2 to 60 Hz or 3 to 50 Hz, or 5 to 40 Hz, for example about 10, 20 or 30Hz. The controller may be configured to modulate the amplitude of the vibration of the membrane with a sinusoidal, saw-tooth or square wave.

The method may further comprise: in step a), periodically performing a scan to determine a resonant frequency of the aerosol generator and / or the membrane; in step b) demodulating the output signal at the scanning frequency; and in step c), determining whether aerosol is present in the channel on the basis of the demodulated output signal.

The method may further comprise, if aerosol is present in the channel, determining the phase difference between the driver signal and the output signal, and thereby determining the velocity of the aerosol as it passes the optical sensor.

### Brief Description of the Figures

Figure 1 shows an expanded view of a vibrating membrane nebulizer.
Figure 2 shows the aerosol generator for the nebulizer of Figure 1.
Figures 3A and 3B are perspective views from either side of the base unit and mouthpiece component of a nebulizer according to the invention.
Figure 4 shows a cut through view of the nebulizer of Figure 3 in the region of the optical sensor.
Figure 5 shows the main components of the optical sensor and the associated region of the channel.
Figure 6 shows the principle of operation of the optical aerosol detection system when aerosol is (Figure 6A) and is not (Figure 6B) present.
Figure 7 shows a simulated output signal from the detector as a function of time as the reservoir becomes empty.
Figure 8 illustrates the principle of amplitude modulation of the driver signal.
Figure 9A shows a simulated output signal from the detector as a function of time as the reservoir becomes empty with a modulated driver signal. Figure 9B shows the output signal from Figure 9A after it has been demodulated.
Figure 10 shows similar graphs to Figure 9, but with simulated external noise added to the output signal.
Figure 11 shows the output signal from the detector as a function of time before, during and after a frequency scan, without (Figure 11A) and with (Figure 11B) additional modulation of the driver signal.
Figure 12 shows the modulation of the driver signal and the resulting output signal from the detector, demonstrating the phase difference between them.

### Detailed Description of the Invention

Nebulizers that detect the presence of aerosol by measuring the amount of light (or other electromagnetic radiation) that is scattered by the droplets are known. For example, US2006/102178 discloses a nebulizer with a mouthpiece having translucent walls, a light transmitter and two light receivers, one for transmitted light and the other for scattered light. With appropriate calibration, the density of the aerosol in the mouthpiece can be determined from the output signals from the receivers. The transmitter may operate intermittently. By subtracting the signal obtained when the transmitter is off from the signal when the transmitter is on, the effect of ambient light can be reduced. US2006/102178 discloses that this method can be used with jet nebulizers or vibrating membrane nebulizers. It does not mention the problem of determining when the membrane becomes dry.

WO2013/042002 discloses a nebulizer that uses a light source and an optical sensor to determine the density of the aerosol by measuring the amount of scattered light. The nebulizer also determines the velocity of the aerosol, by measuring the time delay between the aerosol generator being switched on and the aerosol being detected by the optical sensor. Since the distance from the aerosol generator to the optical sensor is known, the velocity can be calculated. The aerosol generator can be switched on and off in a series of short pulses in order to modulate the average power, and hence the output rate. The nebulizer can be a jet nebulizer, a pressurized metered dose inhaler (pMDI) or a vibrating membrane nebulizer. WO2013/042002 does not mention the problem of determining when the membrane becomes dry.

WO2017/192778 discloses a nebulizer with optical aerosol sensors which measure and detect the presence of droplets within the inhalation channel in order to validate that a dose has been delivered. An LED source emits light which is scattered or absorbed by the droplets, and detected by a photodetector. Multiple light sources and multiple detectors may be used to determine a shape, including a cross-section and length, of the aerosol plume for estimating the ejected mass. However, the nebulizer does not use the detected light signal to determine when the membrane becomes dry. Instead, it monitors changes in the resonance frequency of the membrane.

It has not previously been recognized that this type of optical sensor can be used to reliably detect the absence of liquid in contact with the membrane, by detecting the absence of aerosol within the inhalation channel. In the context of the present invention, the term "optical sensor" means a sensor which detects light, such as visible or infrared light. Similarly, "optical signal" etc. includes both visible and infrared light.

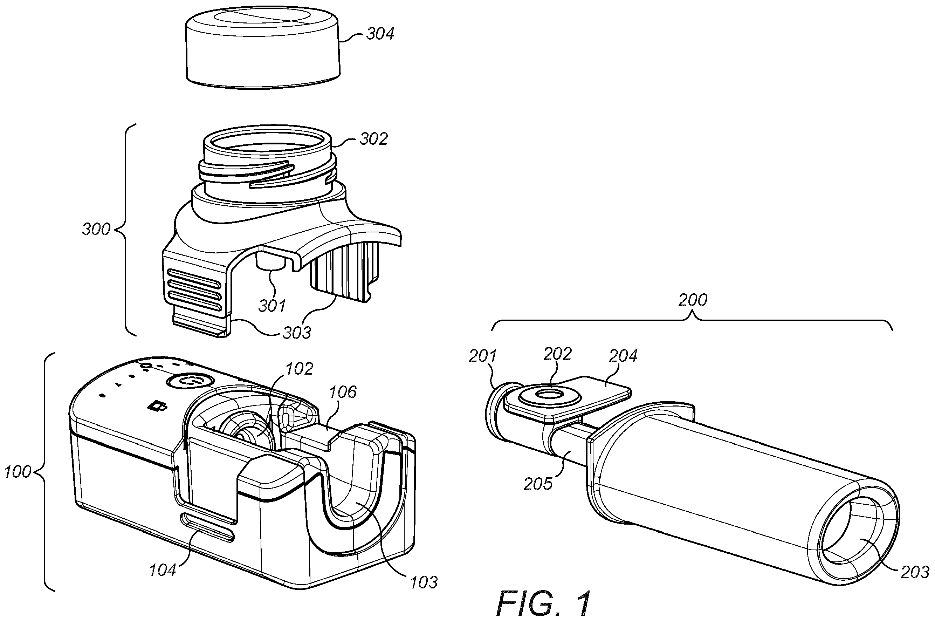

Figure 1 shows an expanded view of a vibrating membrane nebulizer device, which is described in detail in EP2724741 and WO2013/098334. The device comprises three parts: a base unit, a mouthpiece component, and an aerosol head. The base unit **100** has one or more air inlet opening(s), an air outlet opening **102,** a groove **103** for receiving the mouthpiece component **200,** and one or more key lock members **104.** The mouthpiece component **200** has an air inlet opening **201** which is attachable to the air outlet opening **102** of the base unit **100,** a lateral opening **202** for receiving an aerosol generator **301,** and an aerosol outlet opening **203.** A channel **205** extends from the air inlet opening **201** to the aerosol outlet opening. The mouthpiece **200** is insertable into the groove **103** of the base unit **100.** The aerosol head **300** comprises the aerosol generator **301,** a filling chamber **302** for the liquid drug formulation to be aerosolized, which is in fluid contact with the upper end of the aerosol generator **301,** and one or more key lock members **303** complementary to the key lock members **104** of the base unit **100.** A lid **304** closes the filling chamber **302** and prevents contamination or spillage of the liquid during use.

The base unit **100,** the mouthpiece **200** and the aerosol head **300** are detachably connectible with one another. The device is assembled by inserting the mouthpiece **200** into the groove **103** in the base unit **100,** then placing the aerosol head **300** over the mouthpiece **200** and engaging the key lock member(s) **303** of the aerosol head **300** with the complementary member(s) **104** of the base unit **100** by gentle pressure on both the aerosol head and the base unit. The aerosol generator **301** is positioned in the aerosol head **300** in such a way that when engaging the key lock member(s), the aerosol generator **301** is inserted into the lateral opening **202** of the mouthpiece **200.** This creates airtight connections between the aerosol generator **301** and the lateral opening **202** in the mouthpiece as well as between the air outlet opening **102** of the base unit **100** and the air inlet opening **201** of the mouthpiece **200.** The base unit **100,** the mouthpiece **200** and the aerosol head **300** can be separated by reversing these steps.

The base unit **100** may have one or more indentation(s) **106** whose position may be at or near the groove **103,** and the mouthpiece **200** may have one or more positioning member(s) **204.** The indentation(s) of the base unit are complementary to (i.e. shaped to receive) the positioning member (s) **204** of the mouthpiece **200.** In this context, an indentation is a depression (e.g. a recess, pit, cavity, void, notch or the like) whose "negative" shape is complementary to the "positive" shape of a positioning member (which may be a flange, projection, nose, bulge or the like). Together, such indentations and positioning members act to position the mouthpiece correctly in the base unit. The indentation(s) **106** and the positioning member(s) **204** may be asymmetrical, so as to ensure that the mouthpiece **200** can only be inserted into the indentation **106** of the base unit **100** in one particular manner. This ensures that the device is assembled in such a way that the position and orientation of the mouthpiece **200** and base unit **100** relative to each other are correct. The base unit contains a controller, such as a printed circuit board (PCB) which controls the operation of the nebulizer.

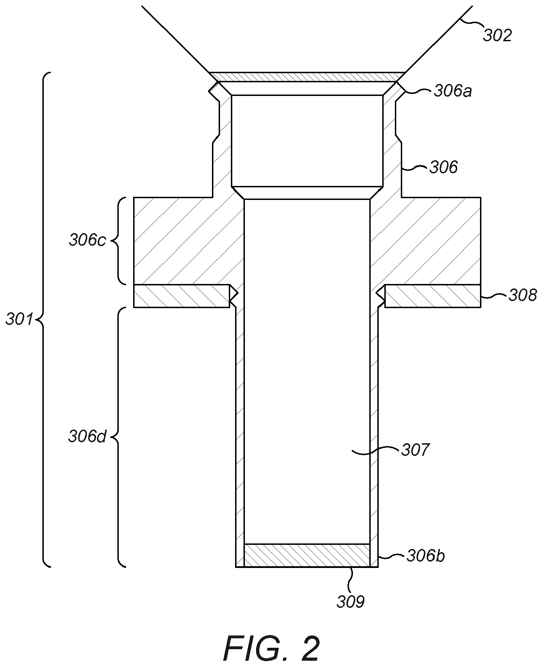

Figure 2 shows the aerosol generator (described in detail in WO2008/058941). It comprises a vibrator, i.e. a piezoelectric element **308,** a transducer body **306** and a membrane **309.** The transducer body **306** is, for example made of stainless steel, titanium or aluminium, and encloses a cavity **307** which contains the liquid to be aerosolized. The inside of the filling chamber **302** is conical so that liquid flows under gravity into the upstream end **306a** of the transducer body **306** and down into the cavity **307.** Together, the filling chamber **302** and cavity **307** form a reservoir for liquid to be aerosolized.

The membrane **309** is positioned at the downstream end **306b** of the transducer body **306.** The holes in the membrane may be formed by electroforming or by laser drilling, with openings normally being in the range from about 1 µm to about 10 µm. Without vibration of the membrane, the balance of pressures, the shape of the holes and the nature of the material used for the membrane are such that the liquid does not seep out through the membrane. However, vibration of the membrane leads to the formation and emission of aerosol droplets through the holes. The membrane may be made of plastic, silicon, ceramic or more preferably metal, and may be affixed onto or into the downstream end **306b** of the aerosol generator **301** by various means, such as gluing, brazing, crimping or laser welding. Optionally, the membrane at least partially forms a dome in its central region, which causes the jet of nascent aerosol droplets to diverge and hence reduces the risk of droplet coalescence.

The piezoelectric element **308** is preferably an annular single or multi-layer ceramic, which vibrates the transducer body **306** in a longitudinal mode. A driver circuit generates the driver signal that excites the piezoelectric element and hence causes the membrane **309** to vibrate, typically at a frequency in the range of 50 - 200 kHz. The frequency of the driver signal may be chosen to be a fixed offset from the resonant frequency of the aerosol generator, for example 500 Hz below it. Excitation of the piezoelectric element causes micronic longitudinal displacements and / or deformations in a direction parallel to the symmetry axis of the transducer body **306.** The transducer body **306** has a region close to the piezoelectric element **308** with a relatively large wall thickness, which serves as a stress concentration zone **306c,** and a region downstream thereof **306d** with a relatively low wall thickness which serves as a deformation amplification zone. In this configuration, the vibrations or deformations of the transducer body **306** caused by the piezoelectric element **308** are amplified. Preferably, the piezoelectric element **308** is located at the level of, or adjacent to, the stress concentration zone **306c.** The internal diameter of the transducer body **306** at the deformation amplification zone **306d** may be the same as at the stress concentration zone **306c,** so that the differences in wall thickness correspond to different external diameters. Alternatively, the external diameter of the transducer body **306** may be constant, while the inner diameters differ at the position of the two zones.

When the nebulizer is operated, aerosol is generated by the membrane **309** and released into the channel **205** where it is mixed with incoming air from the air inlet opening **201** (via the air inlets and air outlet opening **102** of the base unit). The air and aerosol then flow along the channel **205** and out through the an aerosol outlet opening **203** of the mouthpiece and into the patient's airway. Thus the channel **205** is an inhalation channel which provides the pathway for the air and aerosol to the patient.

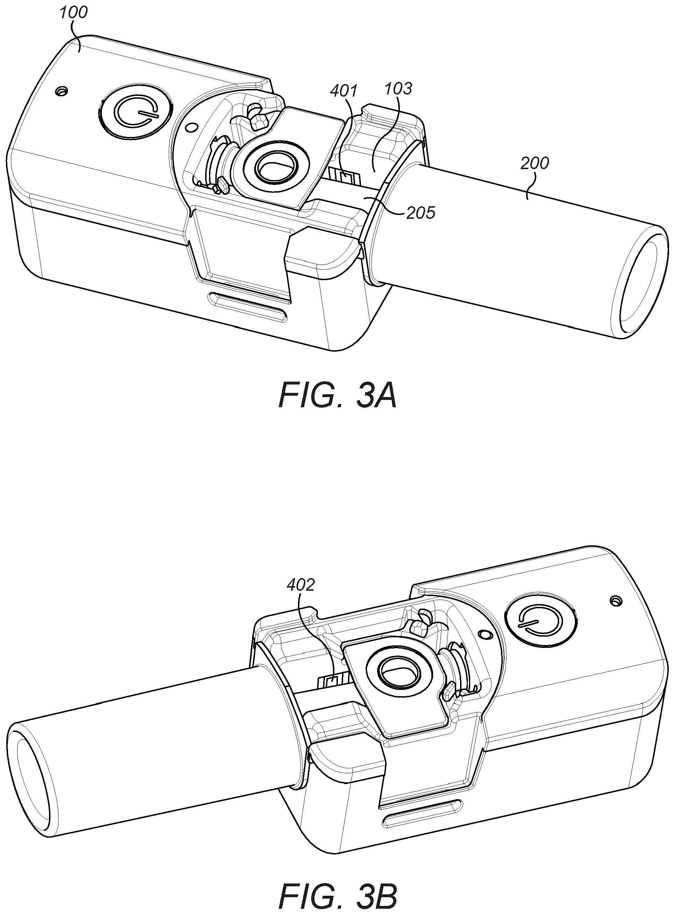

Figures 3A and 3B are perspective views from either side of the base unit **100** and mouthpiece component **200** of a nebulizer according to the invention. The nebulizer is essentially the same as that shown in Figure 1, except that the base unit has windows **401, 402** on either side of the groove **103** downstream of the location of the aerosol generator, at the narrowest part of the channel **205.** The windows **401, 402** may be shaped so as to be lenses.

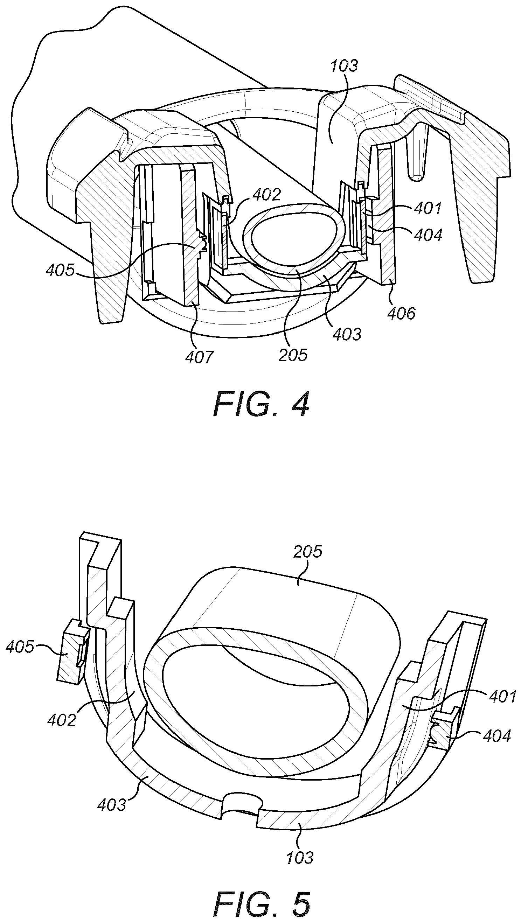

Figure 4 shows a cut through view of this part of the nebulizer, looking downstream, i.e. towards the aerosol exit aperture of the mouthpiece. Figure 5 is a simplified view showing the key components of the optical sensor with the other parts of the base unit and mouthpiece removed for illustration, apart from a section of the channel **205.** A U-shaped bracket **403** forms the base and sides of the groove **103** in this region. The windows **401, 402** are mounted in the bracket **403.** An emitter **404,** such as an Infrared (IR) light emitting diode, is located behind one of the windows **401,** and a detector **405,** such as a photodiode, is located behind the other window **402.** The emitter and detector are each mounted on a PCB **406, 407** respectively. The combination of these components form an optical sensor. They may be standard electronic components, and are chosen so that they operate in the same IR wavelength range, for example 940 nm. The windows are made from a material that is transparent to IR radiation in this range, for example polycarbonate. The windows may be made from a material that acts as an IR filter that is transparent to IR radiation, but does not transmit other wavelengths, such as visible light. The channel **205** is also made from a material that is transparent to IR radiation, for example polypropylene.

Alternatively, it would be possible to use an emitter and detector that operate in a different part of the electromagnetic spectrum, such as visible light. However, using infrared has a number of advantages. Firstly, it is strongly absorbed by aerosols and secondly it cannot distract the patient since it is not visible. This is particularly important if, as described in WO2013/098334, the nebulizer guides the patient by illuminating the mouthpiece with light that increases in intensity as the inhalation approaches the optimal rate.

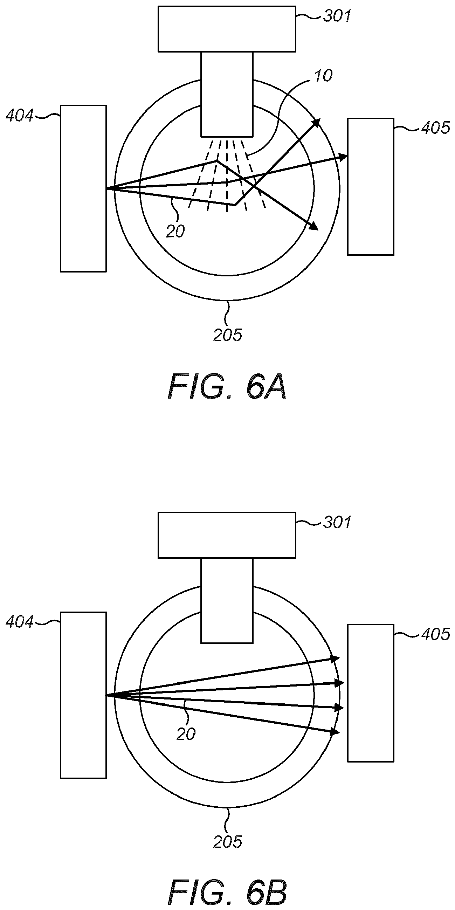

Figure 6 is a schematic showing the basic operation of the optical aerosol detection system. The output signal from the detector depends on the density of the aerosol. When aerosol **10** is produced by the aerosol generator **301,** it is drawn along the channel **205** by the patient's inhalation and into the region between the emitter **404** and the detector **405** which is on the opposite side of the channel. Radiation **20** from the transmitter **404** is scattered by the aerosol **10,** so that only a portion of it is received by the detector **405,** as illustrated in Figure 6A. When the liquid in the reservoir is used up, aerosol is no longer generated, so the amount of radiation received by the detector is increased (Figure 6B). Alternatively, the detector could be positioned so as to receive scattered light instead of transmitted light. For example it could be next to the emitter so that it receives light scattered through approximately 180°, or on the top or bottom of the channel so the it receives light scattered through approximately 90°.

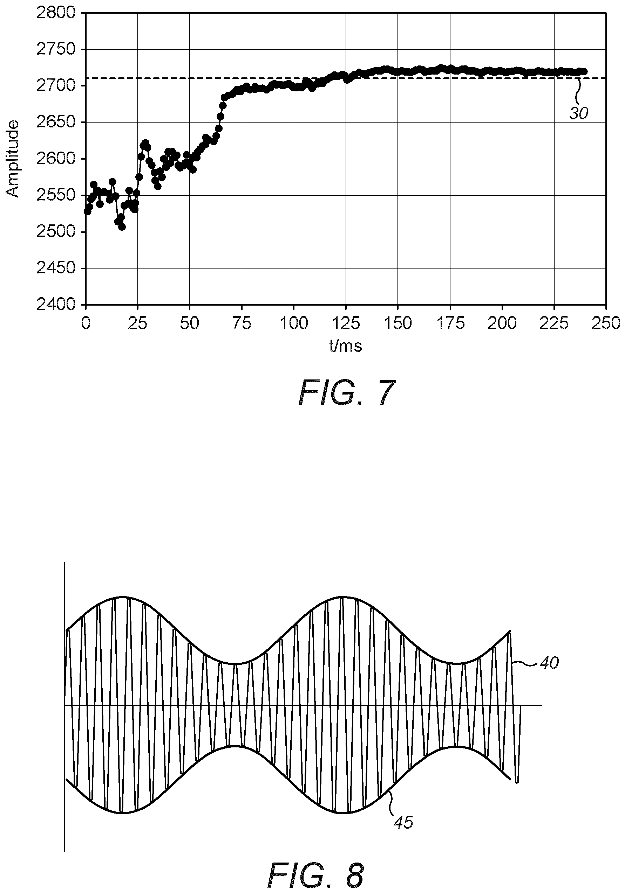

Figure 7 shows a graph of a simulated output signal from the detector (in dimensionless units that represent the light intensity) as a function of time as the reservoir becomes empty. At t=0, there is a small amount of liquid remaining in the reservoir, i.e. the treatment has nearly been completed. At this point aerosol is present in the channel, causing light to be scattered away from the detector, so the signal is low. As the reservoir becomes empty, the amount of aerosol in the channel decreases, and the detector signal increases. The signal fluctuates due to inherent noise from the electronic components (this is apparent, for example between about 20 and 40ms); however external sources of noise (e.g. fluctuations in the ambient light) are not included in this simulated data. At about t = 75ms, the signal begins to plateau, because there is very little aerosol in the channel. After about t = 150ms, the detector signal reaches a maximum; at this point there is no aerosol in the channel. A threshold value (here 2710) is pre-set. When the detector signal exceeds the threshold **30,** the controller determines that the reservoir is empty.

In reality, the detector would pick up ambient background IR radiation, e.g. from sunlight or room lights, which adds noise to the signal. There are also other external sources of noise, such as deposition of aerosol droplets on the inside of the channel near the emitter and / or the detector, or slight changes in the orientation of the mouthpiece relative to the base unit as it is held by the patient. The noise could cause the detector signal to reach the threshold level while aerosol is still present in the channel, and hence result in the aerosol generator being switched off too early. Moreover, there may be small variances between different nebulizers, for example, in the orientation of the emitter and detector as they are soldered onto their PCBs; or there may be slight changes in the optical properties of the channel (in particular the windows) over time, for example as a result of it being repeatedly subjected to heating in order to sterilize the nebulizer between treatments. Since the threshold value for determining whether aerosol is present in the channel is pre-set, it cannot account for these effects. This could also result in an incorrect determination of when the reservoir becomes empty.

The present inventors have identified a method for eliminating, or at least substantially reducing these effects, so that the signal to noise ratio is increased and the sensitivity and reliability of the empty detection method is improved. This is achieved by modulating the amplitude of the driver signal **40,** as shown schematically in Figure 8. The modulation **45** is shown as a sine wave, but may be any kind of periodic signal, for example a saw-tooth wave or a square wave. The amplitude of the driver signal **40** can be modulated at a relatively low frequency, suitably in the range of 1 to 100 Hz, such as about 2 to 70 Hz or 3 to 55 Hz, or 5 to 40 Hz, for example about 10, 20 or 30Hz. However, the mains voltage frequency (which is usually 50 Hz or 60 Hz) should not be used, in order to avoid introducing noise arising from fluctuations in light intensity at mains frequency from mains-powered light sources. While Figure 8 shows a driver signal **40** with a frequency of about 15 times that of the modulation **45** for illustration, in reality the driver signal frequency is typically more like 10,000 times that of the modulation frequency.

The modulation of the driver signal generates a corresponding modulation in the aerosol output rate from the membrane. This in turn creates a modulation in the aerosol density in the channel, and hence in the output signal from the detector. The modulation adds information to the driver signal, which is carried through to the output signal from the detector. This added information is immune to external influences, such as ambient IR radiation, droplet deposition on the inside of the channel, production variances and changes in the optical properties of the mixing channel over the lifetime of the nebulizer.

Modulation of the amplitude of the driver signal need not cause the aerosol output rate to decrease since the root mean square amplitude of the driver signal can be the same as that without modulation. In other words, lower aerosol output when the amplitude is reduced during modulation is balanced by higher aerosol output when the amplitude is increased during modulation.

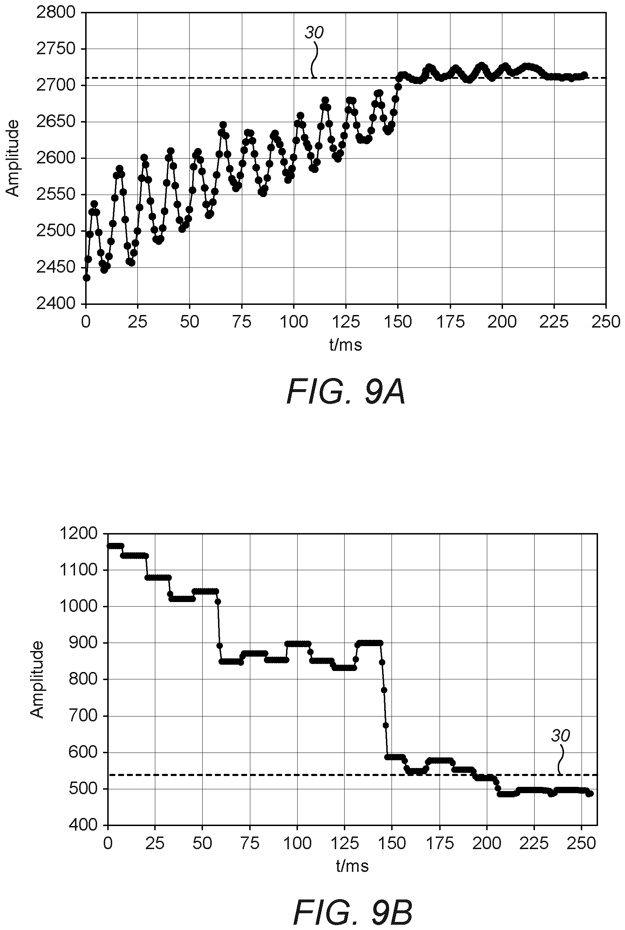

Figure 9A shows a similar graph to Figure 7, but with the driver signal modulated at a frequency of 75 Hz. The modulation in the driver signal produces a corresponding modulation in the detector signal. At about t = 150ms, the detector signal reaches a plateau, at which point there is very little aerosol in the channel. After t = 225ms, the modulation disappears because no aerosol remains, i.e. the reservoir is empty. As with Figure 7, it is possible to determine when the reservoir becomes empty, by comparing the detector signal to a threshold **30.**

The output signal from the detector can be demodulated, for example by digital signal processing, or by an analogue filter (e.g. using electronic components on the detector PCB). Figure 9B shows the demodulated signal from Figure 9A. The y axis is a dimensionless measurement of how closely the output signal corresponds to the modulation as a function of time). When aerosol is present at a relatively high density, i.e. up to about 150ms, the demodulated signal is quite high. Then, as the aerosol density decreases, the demodulated signal decreases. After about 225ms, there is no aerosol present so there are no longer modulations in the output signal. At this point, the demodulated signal reaches a constant minimum value. A threshold **30** in the demodulated signal can also be used to determine that the reservoir is empty; in this case, 550 is a suitable value.

As mentioned above, no external effects are included in the simulated data in Figure 9, and it would be straightforward to determine when the reservoir becomes empty from Figure 9A by using a threshold. However, in reality, there are a number of sources of external noise which can significantly reduce the signal to noise ratio and hence make this method of determining when the reservoir becomes empty rather difficult or unreliable.

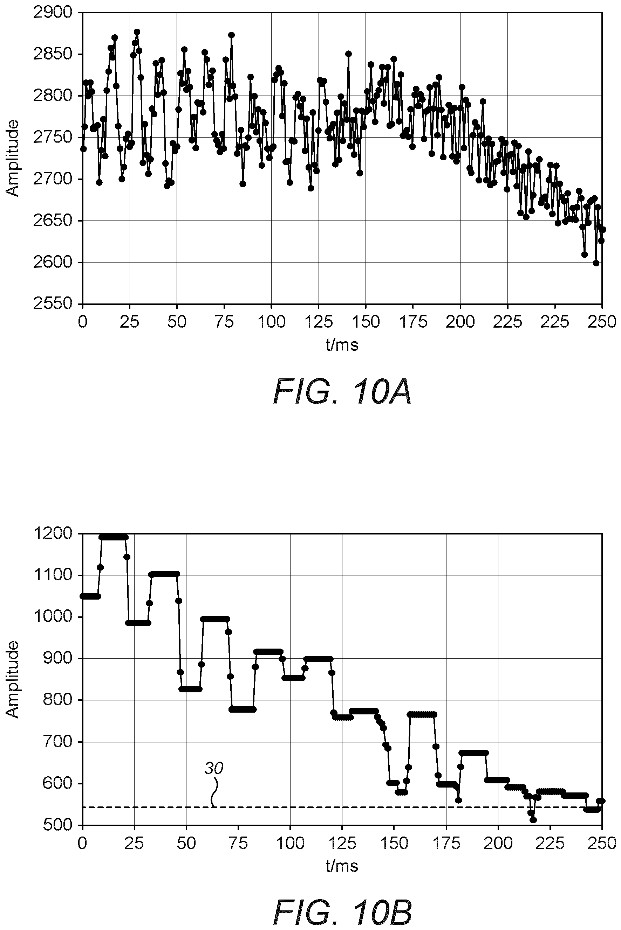

Figure 10A is similar to Figure 9A, but additionally includes simulated external noise, such as fluctuations in the background light intensity (which result in fluctuations in the detector signal) and the effect of aerosol droplet deposition within the channel. As more droplets are deposited during the course of a treatment, there is a gradual increase the amount of light scattered by the deposited droplets, and hence a decrease in the transmitted light. This could be followed by an increase as the deposited droplets coalesce into larger drops which cause less scattering and which may also move down to the bottom of the channel under gravity, so that they are no longer in the path of the transmitted light. Thus, unlike Figure 9A, it is not apparent from Figure 10A whether the reservoir is empty or not.

Figure 10B shows the demodulated signal from Figure 10A. Since the demodulated signal is immune to the external noise that is not at the modulation frequency, the signal is similar to that of Figure 9B. The demodulated signal has an improved signal to noise ratio, and so the point at which the reservoir becomes empty can be determined by applying a threshold **30** with a value of 550, as in Figure 9B. Thus modulating the driver signal results in more accurate information on the amount of IR radiation scattered by the aerosol. This not only increases the sensitivity and reliability of the empty detection method, but also facilitates the determination of the aerosol density.

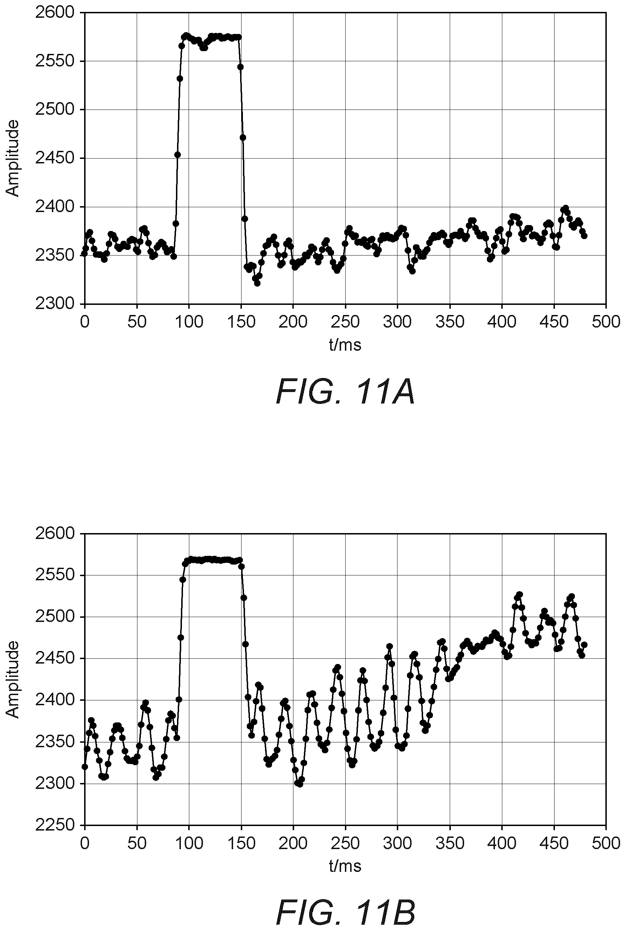

In some vibrating mesh nebulizers, the resonant frequency of the aerosol generator may change during the course of treatment, for example, as the amount of liquid within the reservoir decreases. The frequency of the driver signal may depend on, or be chosen according to, the resonant frequency of the aerosol generator. Consequently, it may be necessary to measure the resonant frequency at intervals throughout operation of the aerosol generator, for example every 0.5 seconds. Typically, in order to determine the resonant frequency, a scan is performed by vibrating the membrane at a series of different frequencies which span the range in which resonance occurs. However, because most of these frequencies are not the optimum driving frequency, the aerosol output rate is inevitably reduced during the period of the scan, which may typically take around 50ms. Figure 11A shows the detector signal over a period of 0.5s. A scan takes place from about t = 100 to 150ms, causing the aerosol output rate to drop almost to zero. The detector signal is substantially higher during this time. Thus the scan process for determining the resonant frequency inherently imposes a square wave-type modulation of the driver signal, in this case 450ms of normal output followed by 50ms of low aerosol output. Performing a scan every 0.5s corresponds to a scanning frequency of 2 Hz.

Figure 11B shows a similar graph to Figure 11A, in which the driver signal is also modulated at 37 Hz. The modulation is not visible in the detector signal from about 100 to 150ms because the aerosol output rate is essentially zero during the scan.

The inherent modulation from the scan could be used instead of the amplitude modulation described above, or in addition to this amplitude modulation as shown in Figure 11B. Of course, the frequency and waveform are fixed by the parameters of the scan. In particular, the scanning frequency needs to be quite low, in order to not to reduce the overall aerosol output rate. Thus the scan modulation has a limited sampling rate, which reduces the information that can be obtained from it.

The modulation of the driver signal can also be used to measure the velocity of the aerosol and the volumetric flow rate. The time that the aerosol takes to travel from the membrane to the optical sensor can be determined by measuring the phase difference between the driver signal and the output signal from the detector. This phase difference can be obtained by cross-correlating the driver and detector signals. The aerosol velocity is then obtained from the calculated time and the known distance between the membrane and the optical sensor. The flow rate can be calculated from the aerosol velocity and the known geometry of the channel.

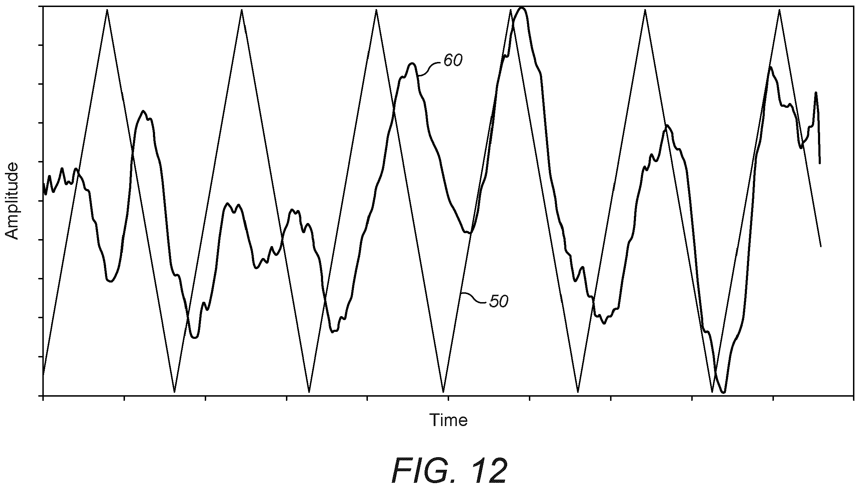

Figure 12 shows a saw-tooth modulation **50** of the driver signal and the resulting detector signal **60,** and illustrates the phase difference between them. The phase difference can be determined using an algorithm which shifts the signals relative to each other and computes the position where there is the most overlap between the signals. This continuously determines the phase difference from a large number of peaks. Thus it is more robust than simply measuring the phase difference between a single peak in each signal, because the phase difference could vary slightly, e.g. if there is any turbulence in the channel.

Using the phase difference to determine the aerosol velocity has the advantage that aerosol generation is not interrupted, so the output rate is not reduced. This contrasts with the method of WO2013/042002 in which the aerosol generator is repeatedly switched on and off to create a series of short bursts in the driver signal, in order to measure the time delay between the start of each burst and the time at which the optical signal decreases as the aerosol droplets arrive at the optical sensor.

The invention can be used in many vibrating membrane nebulizers, for example of the types described in US9027548, WO2012/046220 and WO2015/193432. In these type of nebulizer, the membrane is mounted directly on the piezoelectric element, or has an annular, planar support member on which the membrane and the piezoelectric element are mounted (in contrast to the tubular transducer body described above). However, the invention is especially advantageous in nebulizers of the type described in EP2724741 and WO2013/098334 for a number of reasons.

Firstly, this type of nebulizer is designed to operate with a constant, low flow rate in one direction only. In order to achieve this, it may have a variable flow restrictor that causes less restriction of the flow at a low under-pressure than at a high a under-pressure. Thus, if the patient tries to inhale too hard or fast (i.e. with too much under-pressure), the flow is restricted so that the flow rate does not increase. The nebulizer may be configured to allow the patient to inhale air and/or aerosol through the mouthpiece at an inspiratory flow rate of not more than about 20 L/min, such as from about 10 to 20 L/min, or from about 12 to 18 L/min, such as about 15 L/min. Alternatively, or in addition, the nebulizer may provide the patient with visible, audible or tactile feedback or guidance such as to enable the patient to inhale at the desired inspiratory flow rate. In particular, the nebulizer may provide inhalation effort feedback to the patient by illuminating the mouthpiece with light that increases in intensity as the inhalation approaches the optimal rate. Together, these guide the patient to inhale at a constant, low flow rate. The nebulizer may be breath-actuated, and may have a valve (e.g. just upstream of the air outlet opening of the base unit) which opens and closes at the start and end of each inhalation respectively (for example after a pre-set inhalation time), so that the patient is not able to exhale into the mouthpiece. This ensures that the flow is in one direction only, i.e. towards the aerosol exit aperture.

Secondly, the channel has a small internal volume and is shaped to produce laminar aerosol flow. The internal volume of the channel between the membrane and the optical sensor may be less than 5 cm³ for example from 0.5 to 3 cm³ or from 1 to 2 cm³, such as about 1.5 cm³. In contrast, some vibrating mesh nebulizers have an inhalation channel with a large internal volume, or a chamber in which aerosol is collected. For example, nebulizers which generate aerosol continuously (as opposed to being breath-actuated) may need a large volume in which to store the aerosol that is generated while the patient exhales so that it is not wasted. An inhalation channel which has a large volume between the membrane and the optical sensor would effectively act as a low pass filter for the modulation in the aerosol density. The modulation frequency (^{~} 10 Hz) is too high to pass through a large chamber, and would be averaged to a "continuous" aerosol density. Consequently, the modulation would not be observed in the output signal from the detector. The constant, slow, laminar flow of aerosol past the optical sensor through the small volume channel prevents accumulation of the aerosol. If the aerosol did accumulate in the channel, the modulation of the aerosol density would not be detectable. Moreover laminar flow reduces deposition of the aerosol within the channel, and so reduces a source of noise in the optical signal.

Finally, the channel is part of the mouthpiece component that is detachable from the rest of the nebulizer, and so can easily be made from a material that is transparent to IR and / or visible radiation.

The combination of a slow, one-directional flow rate and a small channel that is separate component is ideal for the method of determining whether there is liquid present on the membrane by measuring a modulated optical signal arising from the aerosol in the channel.

## Claims

1. An inhalation device comprising:
• a channel (205) having an air inlet opening (201) and an aerosol outlet opening (203),
• an aerosol generator (301) comprising a vibrator (308) and a membrane (309),
• a reservoir (302, 307) for liquid to be aerosolized which is fluidically connected to the membrane (309),
• an optical sensor (404, 405) for detecting the presence of aerosol (10) within the channel (205),
• a controller which is configured (i) to provide a driver signal (40) to operate the vibrator so that the membrane (309) vibrates and generates an aerosol in the channel, and to modulate the amplitude of the driver signal at a frequency of from 1 to 100 Hz; (ii) to receive an output signal from the sensor and to demodulate the output signal; (iii) to determine whether aerosol is present in the channel on the basis of the demodulated output signal; and (iv) to stop operating the vibrator if it determines that no aerosol is present.

2. An inhalation device according to claim 1, wherein the optical sensor (404, 405) operates in the infrared region.

3. An inhalation device according to claim 1 or 2, wherein the optical sensor comprises an emitter (404) and a detector (405) that are arranged on opposite sides of the channel (205).

4. An inhalation device according to any of claims 1 to 3, wherein the controller is configured to modulate the amplitude of the driver signal at a frequency of from 5 to 40 Hz, such as about 10 Hz.

5. An inhalation device according to any of claims 1 to 4, wherein the controller is configured (i) to periodically perform a scan to determine a resonant frequency of the aerosol generator and / or the membrane; (ii) to demodulate the output signal at a scanning frequency that corresponds to the period between scans; and (iii) to determine whether aerosol is present in the channel on the basis of the demodulated output signal.

6. An inhalation device according to any of claims 1 to 5, wherein the controller is configured to determine a phase difference between the modulated driver signal and the output signal, and thereby to determine the velocity of the aerosol.

7. An inhalation device according to any of claims 1 to 6, which further comprises a variable flow restrictor which restricts the flow rate of the air and aerosol to a maximum flow rate of about 20 L/min.

8. An inhalation device according to any of claims 1 to 7, further comprising a sensor for measuring pressure in the channel and a signalling device capable of emitting light of varying intensity, wherein the controller is configured (i) to receive a signal representing the measured pressure and (ii) to cause the signalling device to emit light of lower intensity the further the measured pressure deviates from a target pressure.

9. An inhalation device according to any of claims 1 to 8, wherein the channel (205) has an internal volume between the membrane (309) and the optical sensor (404, 405) of less than 5 cm³.

10. An inhalation device according to any of claims 1 to 9, wherein the channel (205) is part of a component (200) that is removable from the rest of the device.

11. A method of operating an inhalation device according to any of claims 1 to 10, the method comprising:
a) providing a driver signal to operate the vibrator so that the membrane vibrates and generates an aerosol in the channel, and modulating the amplitude of the driver signal at a frequency of from 1 to 100 Hz;
b) receiving an output signal from the optical sensor and demodulating the output signal at the same frequency;
c) determining whether aerosol is present in the channel on the basis of the demodulated output signal; and
d) ceasing to operate the vibrator if it is determined in step c) that no aerosol is present.

12. A method according to claim 11, wherein the amplitude of the driver signal is modulated at a frequency of from 5 to 40 Hz, such as about 10 Hz.

13. A method according to claim 11 or claim 12, wherein the amplitude of the driver signal is modulated with a sinusoidal, saw-tooth or square wave.

14. A method according to any of claims 11 to 13, the method further comprising: in step a), periodically performing a scan to determine a resonant frequency of the aerosol generator and / or the membrane; in step b) demodulating the output signal at the scanning frequency; and in step c), determining whether aerosol is present in the channel on the basis of the demodulated output signal.

15. A method according to any of claims 11 to 14, the method further comprising measuring the phase difference between the modulated driver signal and the output signal, and thereby determining the velocity of the aerosol.

## Patentansprüche

1. Inhalationsvorrichtung, die Folgendes umfasst:
• einen Kanal (205), der eine Lufteinlassöffnung (201) und eine Aerosolauslassöffnung (203) aufweist,
• einen Aerosolgenerator (301), der einen Schwingungserzeuger (308) und eine Membran (309) umfasst,
• einen Behälter (302, 307) für eine Flüssigkeit, die aerosolisiert werden soll, der mit der Membran (309) strömungstechnisch verbunden ist,
• einen optischen Sensor (404, 405) zum Detektieren des Vorhandenseins eines Aerosols (10) im Kanal (205) und
• eine Steuereinheit, die konfiguriert ist zum (i) Bereitstellen eines Ansteuersignals (40), um den Schwingungserzeuger derart zu betreiben, dass die Membran (309) in Schwingungen versetzt wird und ein Aerosol im Kanal erzeugt, und Modulieren der Amplitude des Ansteuersignals bei einer Frequenz im Bereich von 1 bis 100 Hz; (ii) Empfangen eines Ausgangssignals vom Sensor und Demodulieren des Ausgangssignals; (iii) Bestimmen, ob Aerosol im Kanal vorhanden ist, auf der Grundlage des demodulierten Ausgangssignals; und (iv) Stoppen des Betriebs des Schwingungserzeugers, falls bestimmt wird, dass kein Aerosol vorhanden ist.

2. Inhalationsvorrichtung nach Anspruch 1, wobei der optische Sensor (404, 405) im Infrarotbereich arbeitet.

3. Inhalationsvorrichtung nach Anspruch 1 oder 2, wobei der optische Sensor einen Strahler (404) und einen Detektor (405) umfasst, die auf gegenüberliegenden Seiten des Kanals (205) angeordnet sind.

4. Inhalationsvorrichtung nach einem der Ansprüche 1 bis 3, wobei die Steuereinheit konfiguriert ist, die Amplitude des Ansteuersignals bei einer Frequenz im Bereich von 5 bis 40 Hz, z. B. etwa 10 Hz, zu modulieren.

5. Inhalationsvorrichtung nach einem der Ansprüche 1 bis 4, wobei die Steuereinheit konfiguriert ist zum (i) Durchführen einer Abtastung in regelmäßigen Abständen, um eine Resonanzfrequenz des Aerosolgenerators und/oder der Membran zu bestimmen; (ii) Demodulieren des Ausgangssignals bei einer Abtastfrequenz, die dem Zeitraum zwischen Abtastungen entspricht; und (iii) Bestimmen, ob Aerosol im Kanal vorhanden ist, auf der Grundlage des demodulierten Ausgangssignals.

6. Inhalationsvorrichtung nach einem der Ansprüche 1 bis 5, wobei die Steuereinheit konfiguriert ist, eine Phasendifferenz zwischen dem modulierten Ansteuersignal und dem Ausgangssignal zu bestimmen und dadurch die Geschwindigkeit des Aerosols zu bestimmen.

7. Inhalationsvorrichtung nach einem der Ansprüche 1 bis 6, die ferner einen variablen Durchflussregulierer umfasst, der die Durchflussmenge der Luft und des Aerosols auf eine maximale Durchflussmenge von etwa 20 l/min beschränkt.

8. Inhalationsvorrichtung nach einem der Ansprüche 1 bis 7, die ferner einen Sensor zum Messen eines Drucks im Kanal und eine Signalisierungsvorrichtung, die Licht variierender Intensität abstrahlen kann, umfasst, wobei die Steuereinheit konfiguriert ist zum (i) Empfangen eines Signals, das den gemessenen Druck repräsentiert, Signalisierungsvorrichtung Licht einer niedrigeren Intensität abstrahlt, je weiter der gemessene Druck von einem Zieldruck abweicht.

9. Inhalationsvorrichtung nach einem der Ansprüche 1 bis 8, wobei der Kanal (205) ein Innenvolumen zwischen der Membran (309) und dem optischen Sensor (404, 405) von weniger als 5 cm³ aufweist.

10. Inhalationsvorrichtung nach einem der Ansprüche 1 bis 9, wobei der Kanal (205) Teil einer Komponente (200) ist, die vom Rest der Vorrichtung abnehmbar ist.

11. Verfahren zum Betreiben einer Inhalationsvorrichtung nach einem der Ansprüche 1 bis 10, wobei das Verfahren Folgendes umfasst:
a) Bereitstellen eines Ansteuersignals, um den Schwingungserzeuger derart zu betreiben, dass die Membran in Schwingungen versetzt wird und ein Aerosol im Kanal erzeugt, und Modulieren der Amplitude des Ansteuersignals bei einer Frequenz im Bereich von 1 bis 100 Hz;
b) Empfangen eines Ausgangssignals vom optischen Sensor und Demodulieren des Ausgangssignals bei derselben Frequenz;
c) Bestimmen, ob Aerosol im Kanal vorhanden ist, auf der Grundlage des demodulierten Ausgangssignals; und
d) Beenden des Betriebs des Schwingungserzeugers, wenn in Schritt c) bestimmt wird, dass kein Aerosol vorhanden ist.

12. Verfahren nach Anspruch 11, wobei die Amplitude des Ansteuersignals bei einer Frequenz im Bereich von 5 bis 40 Hz, z. B. etwa 10 Hz, moduliert wird.

13. Verfahren nach Anspruch 11 oder Anspruch 12, wobei die Amplitude des Ansteuersignals mit einer Sinus-, Sägezahn- oder Rechteckschwingung moduliert wird.

14. Verfahren nach einem der Ansprüche 11 bis 13, wobei das Verfahren ferner Folgendes umfasst: in Schritt a) Durchführen einer Abtastung in regelmäßigen Abständen, um eine Resonanzfrequenz des Aerosolgenerators und/oder der Membran zu bestimmen; in Schritt b) Demodulieren des Ausgangssignals bei der Abtastfrequenz und in Schritt c) Bestimmen, ob Aerosol im Kanal vorhanden ist, auf der Grundlage des demodulierten Ausgangssignals.

15. Verfahren nach einem der Ansprüche 11 bis 14, wobei das Verfahren ferner ein Messen der Phasendifferenz zwischen dem modulierten Ansteuersignal und dem Ausgangssignal und dadurch ein Bestimmen der Geschwindigkeit des Aerosols umfasst.

## Revendications

1. Dispositif d'inhalation comprenant :
• un canal (205) ayant une ouverture d'entrée d'air (201) et une ouverture de sortie d'aérosol (203),
• un générateur d'aérosol (301) comprenant un vibreur (308) et une membrane (309),
• un réservoir (302, 307) pour du liquide à pulvériser qui est relié de manière fluidique à la membrane (309),
• un capteur optique (404, 405) pour détecter la présence d'aérosol (10) à l'intérieur du canal (205),
• un dispositif de commande qui est configuré (i) pour fournir un signal de commande (40) pour faire fonctionner le vibreur de sorte que la membrane (309) vibre et génère un aérosol dans le canal, et pour moduler l'amplitude du signal de commande à une fréquence de 1 à 100 Hz ; (ii) pour recevoir un signal de sortie provenant du capteur et pour démoduler le signal de sortie ; (iii) pour déterminer si un aérosol est présent dans le canal sur la base du signal de sortie démodulé ; et (iv) pour arrêter le fonctionnement du vibreur s'il détermine qu'aucun aérosol n'est présent.

2. Dispositif d'inhalation selon la revendication 1, dans lequel le capteur optique (404, 405) fonctionne dans la région infrarouge.

3. Dispositif d'inhalation selon la revendication 1 ou 2, dans lequel le capteur optique comprend un émetteur (404) et un détecteur (405) qui sont agencés sur des côtés opposés du canal (205).

4. Dispositif d'inhalation selon l'une quelconque des revendications 1 à 3, dans lequel le dispositif de commande est configuré pour moduler l'amplitude du signal de commande à une fréquence de 5 à 40 Hz, telle qu'environ 10 Hz.

5. Dispositif d'inhalation selon l'une quelconque des revendications 1 à 4, dans lequel le dispositif de commande est configuré (i) pour effectuer périodiquement un balayage pour déterminer une fréquence de résonance du générateur d'aérosol et/ou de la membrane ; (ii) pour démoduler le signal de sortie à une fréquence de balayage qui correspond à la période entre des balayages ; et (iii) pour déterminer si un aérosol est présent dans le canal sur la base du signal de sortie démodulé.

6. Dispositif d'inhalation selon l'une quelconque des revendications 1 à 5, dans lequel le dispositif de commande est configuré pour déterminer une différence de phase entre le signal de commande modulé et le signal de sortie, et ainsi déterminer la vitesse de l'aérosol.

7. Dispositif d'inhalation selon l'une quelconque des revendications 1 à 6, qui comprend en outre un limiteur de débit variable qui limite le débit de l'air et de l'aérosol à un débit maximal d'environ 20 L/min.

8. Dispositif d'inhalation selon l'une quelconque des revendications 1 à 7, comprenant en outre un capteur pour mesurer la pression dans le canal et un dispositif de signalisation capable d'émettre de la lumière d'intensité variable, dans lequel le dispositif de commande est configuré (i) pour recevoir un signal représentant la pression mesurée et (ii) pour amener le dispositif de signalisation à émettre de la lumière d'intensité inférieure au fur et à mesure que la pression mesurée s'écarte d'une pression cible.

9. Dispositif d'inhalation selon l'une quelconque des revendications 1 à 8, dans lequel le canal (205) a un volume interne entre la membrane (309) et le capteur optique (404, 405) inférieur à 5 cm³.

10. Dispositif d'inhalation selon l'une quelconque des revendications 1 à 9, dans lequel le canal (205) fait partie d'un composant (200) qui peut être retiré du reste du dispositif.

11. Procédé de fonctionnement d'un dispositif d'inhalation selon l'une quelconque des revendications 1 à 10, le procédé comprenant :
a) la fourniture d'un signal de commande pour faire fonctionner le vibreur de telle sorte que la membrane vibre et génère un aérosol dans le canal, et la modulation de l'amplitude du signal de commande à une fréquence de 1 à 100 Hz ;
b) la réception d'un signal de sortie provenant du capteur optique et la démodulation du signal de sortie à la même fréquence ;
c) le fait de déterminer si un aérosol est présent dans le canal sur la base du signal de sortie démodulé ; et
d) l'arrêt du fonctionnement du vibreur s'il est déterminé à l'étape c) qu'aucun aérosol n'est présent.

12. Procédé selon la revendication 11, dans lequel l'amplitude du signal de commande est modulée à une fréquence de 5 à 40 Hz, telle qu'environ 10 Hz.

13. Procédé selon la revendication 11 ou la revendication 12, dans lequel l'amplitude du signal de commande est modulée avec une onde sinusoïdale, en dents de scie ou carrée.

14. Procédé selon l'une quelconque des revendications 11 à 13, le procédé comprenant en outre : à l'étape a), la réalisation périodique d'un balayage pour déterminer une fréquence de résonance du générateur d'aérosol et/ou de la membrane ; à l'étape b) la démodulation du signal de sortie à la fréquence de balayage ; et à l'étape c), le fait de déterminer si un aérosol est présent dans le canal sur la base du signal de sortie démodulé.

15. Procédé selon l'une quelconque des revendications 11 à 14, le procédé comprenant en outre la mesure de la différence de phase entre le signal de commande modulé et le signal de sortie, et ainsi la détermination de la vitesse de l'aérosol.
